# EUROPEAN PATENT SPECIFICATION

(11) **EP 4 077 259 B1**
(45) Date of publication and mention of the grant of the patent: **31.01.2024**
(21) Application number: 20821236.5
(22) Date of filing: 14.12.2020
(51) Int. Cl.: C07C 46/06, C07C 37/07, C07C 39/08, C07C 50/02

(54) **FORMATION OF 2,3,5-TRIMETHYLHYDROQUINONE FROM 2,3,6-TRIMETHYLPHENOL**
HERSTELLUNG VON 2,3,5-TRIMETHYLHYDROCHINON AUS 2,3,6-TRIMETHYLPHENOL
FORMATION DE 2,3,5-TRIMÉTHYLHYDROQUINONE À PARTIR DE 2,3,6-TRIMÉTHYLPHÉNOL

(30) Priority: 19.12.2019 EP 19217786
(43) Date of publication of application: 26.10.2022
(73) Proprietor: DSM IP Assets B.V., 6411 TE Heerlen (NL)
(72) Inventor: BONRATH, Werner, 4303 Kaiseraugst (CH); NETSCHER, Thomas, 4303 Kaiseraugst (CH); SCHNEIDER, Michael, 4303 Kaiseraugst (CH); MEDLOCK, Jonathan Alan, 4303 Kaiseraugst (CH)
(74) Representative: dsm-firmenich IP
(86) International application number: PCT/EP2020/085941
(87) International publication number: WO 2021/122437

(56) References cited:
- EP-A1- 0 264 823
- WO-A1-2015/000767
- DE-A1- 2 250 066
- BENJAMIN SCHÄFFNER ET AL: "Organic Carbonates as Solvents in Synthesis and Catalysis", CHEMICAL REVIEWS, vol. 110, no. 8, 11 August 2010 (2010-08-11), pages 4554-4581, XP055618498, US ISSN: 0009-2665, DOI: 10.1021/cr900393d

## Description

### Technical Field

The present invention relates to the synthesis of 2,3,5-trimethylhydroquinone (=TMHQ) from 2,3,6-trimethylphenol (=TMP).

### Background of the invention

2,3,5-trimethylhydroquinone (TMHQ) is a key chemical in the synthesis of α-tocopherol. Typically, 2,3,5-trimethylhydroquinone is obtained by reduction of 2,3,5-trimethylquinone (TMQ), which can be obtained by oxidation of 2,3,6-trimethylphenol (=TMP).

DE 2 221 624 describes the oxidation of 2,3,6-trimethylphenol with oxygen in the presence of copper halides, primarily copper (II) chloride dihydrate, in polar solvents which are water soluble or of unlimited miscibility with water- preferably dimethylformamide. A disadvantage of this process, however, is that it is difficult to isolate the product from the reaction mixture and to recycle the catalyst.

WO 2015/000767 A1 discloses the oxidation of TMP by oxygen in water and an aliphatic alcohol having 4 to 10 carbon atoms in the presence of a copper halide. This process uses two phases, one of which is the aqueous phase.

The reduction of TMHQ by catalytic hydrogenation in presence of an organic solvent is well known. For example, DE 683 908 describes methanol and ethanol as solvent. These solvents, however, lead to autoxidation of TMHQ and undesired coloration and, hence, are considered as being unsuitable solvents for this reaction. Using higher aliphatic alcohols such those mentioned in WO 2015/000767 A1, or unpolar solvents such as toluene, TMHQ, the product of hydrogenation, is precipitated and leads to incrustation which increase cost for maintenance of the reaction equipment as well as for the regeneration of the hydrogenation catalyst. DE 1 940 386 solves this problem by using an aliphatic alcohol with 3 to 5 carbon atoms as solvent and using a temperature of 60 - 180°C.
EP 0 264 823 A1 proposes other solvents such as ketones, organic acids, organic acid esters, ethers, cyclic ethers, and lactones as solvents for the hydrogenation of solvents. However, these solvents have deficiencies in view of environmental aspects, chemical stability towards acids and/or bases and toxicology or fire hazards. DE 22 50 066 A1 discloses the reduction of 2,3,5-trimethylbenzoquone with hydrogen in the presence of a hydrogenation catalyst in a carbonic acid ester with 2 to 12 carbon atoms.

Cyclic alkyl carbonates (=alkylene carbonates) are, somehow, still unusual solvents for chemical reactions, the more and more, however, these solvents are used due to excellent ecotoxicity and positive fire and working hazard properties. Finally, alkylene carbonates are attractive in view of cost.

However, as discussed in US 2003/0060383 A1, an unfortunate property of alkylene carbonates which is well-known to those skilled in the art, is that these compounds are prone to hydrolysis. The hydrolysis leads to formation of CO₂ and the respective alkanediol. Furthermore, it is also known to the person skilled in the art, for example from P. Ziosi et al., Catal. Sci. Technol. 2014, 4, 4386-4395, that phenol and alkylene carbonates form different alkanediol ethers of the phenols.

### Summary of the invention

Therefore, the problem to be solved by the present invention is to offer a process for the manufacturing of TMHQ from TMP, which allows to perform the oxidation step and the reduction step in one suitable solvent which avoids precipitation of the products and intermediates.

It has been, surprisingly, found that a process according to claim 1 is able to solve this problem.

It has been particularly found that the alkylene carbonates of formula (X) are optimally suited for this purpose. Not only remarkably high yields for both reaction steps a) and b) have been found, it has also been observed no indication of any degradation of the solvent during oxidation or reduction steps.

Due to the good solubility of TMP, TMQ and TMHQ the synthesis can be performed at moderate temperatures, which reduce the formation of side or degradation products.

Particularly advantageous in the present process is that the use of different reaction solvents for the subsequent steps can be avoided, which results in less cost for storage equipment (such as additional tanks), less cost for handling of reaction mixtures and less cost for recycling of solvents. Finally, any removal of solvents necessary in the state-of-the-art processes results in additional cost in time and energy used for such operations such as distillation which can be saved in the present process.

Further aspects of the invention are subject of further independent claims. Particularly preferred embodiments are subject of dependent claims.

### Detailed description of the invention

In a first aspect the present invention relates to a process for the manufacturing the compound of the formula (I) comprising the steps reaction step a) followed by reaction step b)
a) oxidation of a compound of the formula (II) to yield a compound of the formula (III); 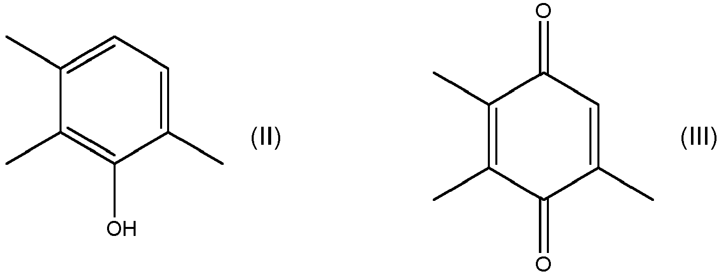
b) reduction of the compound of the formula (III) to yield the compound of the formula (I) 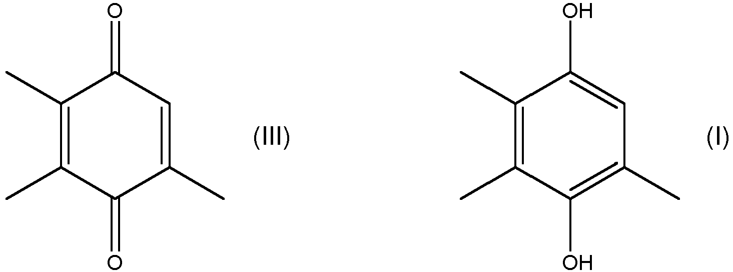 characterized in that both reaction steps a) and b) are performed in at least one solvent of the formula (X) 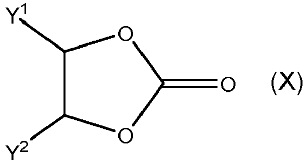 wherein Y¹ and Y² represent independently from each other either H or a methyl or ethyl group;
   or in a two-phase solvent mixture comprising at least one solvent (**A**) being of formula (X) and at least one solvent (**B**) which is not miscible with solvent of formula (X) at room temperature in a volume ratio of (**A**)/(**B**) of between 1/50 and 50/1.

For sake of clarity, some terms as been used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group. Hence, "propyl" can be "*n*-propyl" or "*iso*-propyl" (=isopropyl). Analogously, "butyl" can be "*n*-butyl" or "*iso*-butyl" or "*sec*-butyl" or "*tert*-butyl".

The term "independently from each other" in this document means, in the context of substituents, moieties, or groups, that identically designated substituents, moieties, or groups can occur simultaneously with a different meaning in the same molecule.

In case identical labels for symbols or groups are present in several formulae, in the present document, the definition of said group or symbol made in the context of one specific formula applies also to other formulae which comprises the same said label.

The term "inert", as used in this document in describing a material, means that under the conditions of the reaction said material does not undergo any chemical reaction.

### Step a) oxidation

The process comprises a step a)
a) oxidation of a compound of the formula (II) to yield a compound of the formula (III).

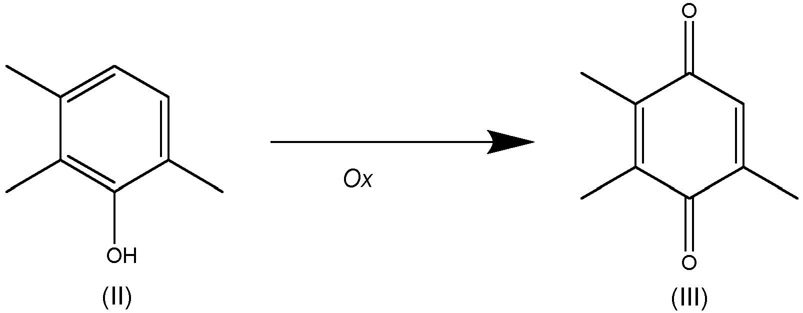

The compound of formula (II), 2,3,6-trimethylphenol (=TMP), is known to the person skilled in the art.

The oxidation is performed by a suitable oxidizing agent (*Ox*). Particularly suitable as oxidizing agent is O₂ or peroxides, particularly in the form of H₂O₂, hydroperoxides, peroxy acids and esters of peroxy acids, in the presence of a catalyst.

It is preferred that the oxidation in step a) is performed using an oxidizing agent which is either oxygen or hydrogen peroxide, more preferred oxygen, in the presence of a catalyst.

Particularly the oxidation is performed in the presence of catalyst being a salt or complex of a transition metal which is selected from the group consisting of Ce, Mn, Fe, Cu and Zn, particularly is selected from the group consisting of Mn, Fe, Cu and Zn, more preferably of Mn(II), Mn(IV), Fe(II), Fe(III), Cu(I), Cu(II) or Zn(II). Particularly the oxidation is preferably performed in the presence of at least one Cu(I) salt and/or at least one Cu(II) salt, more preferred at least one Cu(II) salt. The Cu(II) salts are particularly copper halides. Most preferred is CuCl₂ (this salt is typically used in the form of its hydrate CuCl₂·2H₂O).

Step a) is usually carried out at a temperature of from 40°C to 95°C, particularly between 50°C and 85°C, preferably between 55°C and 80°C.

The above process is preferably carried out in the presence of an organic acid. Preferably, the organic acid is selected from the group consisting of acetic acid, adipic acid, lactic acid, oxalic acid, citric acid, particularly acetic acid.

The amount of organic acid added is in an amount that the pH of the reaction mixture is in the range of 4.8 -6.8, particularly 5 - 6.8, preferably 5.5 - 6.2.

It is preferred that the amounts of organic acid added is 0.15 - 3.5 mol-equivalent, preferably 1.5 -2.5 mol-equivalent, based on the amount of compound of formula (II).

It is important to stress that the optional organic acid is used in small amounts only. This is very advantageous as in view of handling, particularly in view of flammability. The state-of-the-art technology, however, uses large amounts of highly volatile organic substances, such as solvents or organic acids, in the presence of oxygen or oxygen-containing gas mixtures.

Furthermore, when the reaction would be made under strongly acid condition, major quantities of base would be needed when neutralizing the residues after termination of the reaction, which would be, therefore also very disadvantageous from an economic and/or environmental point of view. With the present invention, it has been observed that the reaction excellently proceeds also at conditions at slightly acidic pH.

Therefore, it is preferred that the oxidation takes place at a pH of 5 - 7.8, particularly of 5.5 - 6.8.

In one embodiment, molecular oxygen (O₂) can be used as oxidizing agent. In another embodiment, a mixture comprising oxygen and an inert gas is used as oxidizing agent. It is preferred that the amount of oxygen in such a mixture comprising oxygen and an inert gas is at least 15 % by volume, particularly at least 20 % by volume. Such a mixture may, for example, be a binary mixture such as a mixture oxygen/nitrogen or oxygen/argon or alike. Said mixture can consist of or comprise two or more inert gases. It is particularly preferred to use air as such a mixture comprising oxygen and an inert gas.

It is preferred that that the oxidizing agent is O₂ in the form of air or pure oxygen. Most preferred the oxidizing agent is O₂ in the form of pure oxygen.

Oxygen and particularly air are very cheap oxidizing agents.

The conditions for the oxidation step are, principally, known to the person skilled in the art. If desired the reaction mixture after the step a) can be worked up, particularly extracted.

### Step b) reduction

The process comprises a step b)
b) reduction of the compound of the formula (III) to yield the compound of the formula (I).

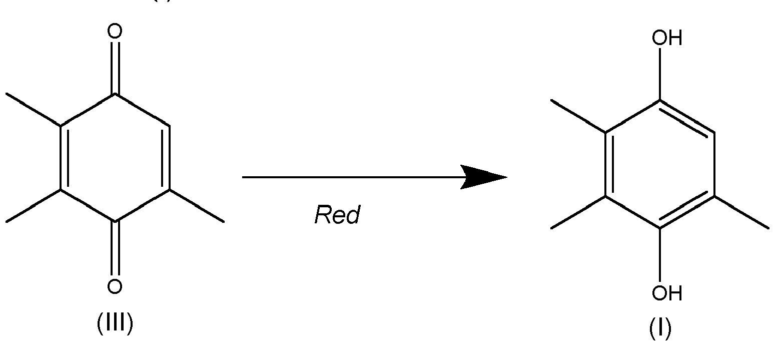

The compound of the formula (III) is reduced in the above process to the compound of the formula (I) by a reducing agent (Red).

In one preferred embodiment of the invention the reducing agent is molecular hydrogen. In other words, in one embodiment the compound of the formula (III) is hydrogenated by molecular hydrogen in the presence of a metal catalyst.

The metal of the metal catalyst is preferably selected from the group consisting of Ni, Ir, Pd, Pt, Rh and Ru, particularly Pd or Pt, particularly a heterogeneous metal catalyst on a carrier or a support material.

The metal catalyst can be a catalyst comprising more than one of the mentioned metals.

It is preferred that the metal of the metal catalyst is palladium.

The metal catalyst is preferably a heterogeneous metal catalyst on a carrier or a support material.

Such carrier material is particularly a solid material having a high surface area, to which the metal is affixed. The support may be inert or participate in the catalytic reactions.

Typical supports/carrier material include various kinds of carbon or an oxide of Si, Al, Ce, Ti or Zr, particularly of Al or Si, such as alumina or silica. The preferred support/carrier material is carbon.

In case the support is Cer, the preferred oxide is CeO₂. Preferably, the oxide of Al is Al₂O₃ and AlO(OH). Particularly preferred is Boehmite.

The surface area of the carrier is preferably in the range of 800 to 1500 m²/g, particularly of 1000 to 1200 m²/g.

The heterogeneous metal catalyst may also be affixed or immobilized on a surface of a larger object typically in form of a structured packing element which might be a part of the reactor in which the reduction takes place or an element which is inserted into said reactor.

The support material is, hence, preferably a structured packing element. This structured packing element may be a dumped packing, a knit, an open-celled foam structure, preferably made of plastic, for example polyurethane or melamine resin, or ceramic, or a structured packing element, as already known in principle, i.e. by its geometric shape, from distillation and extraction technology. However, for the purposes of the present invention, structured packings in principle have a substantially smaller hydraulic diameter, frequently by a factor of from 2 to 10, than comparable internals in the field of distillation and extraction technology. Useful structured packing elements are in particular metal fabric packings and wire fabric packings, for example of the design Montz A3, Sulzer BX, DX and EX. Instead of metal fabric packings, it is also possible to use structured packings made of other woven, knitted or felted materials. Further useful structured packings are of flat or corrugated sheets, preferably without perforation, or other relatively large orifices, for example corresponding to the designs Montz BI or Sulzer Mellapak. The structured packings made of expanded metal are also advantageous, for example structured packings of the type Montz BSH.

It is preferred that the metal catalyst is a palladium catalyst, particularly a palladium on carbon catalyst (Pd/C).

The catalytic metal loading (weight ratio metal/catalyst, i.e. the weight metal / weight (metal+carrier)) is typically between 1 to 20%, preferably between 4 and 11%, more preferably between 4 and 6% by weight. A very preferred heterogeneous metal catalyst is palladium on carbon catalyst (Pd/C) of which 5 % by weight is palladium (i.e. loading = 5%).

In another preferred embodiment of the invention the reducing agent is a transfer hydrogenation agent. In other words, in another embodiment the compound of the formula (III) is transfer hydrogenated by a transfer hydrogenation agent to yield the compound of the formula (I). Said transfer hydrogenation agent is preferably formic acid and/or a formic acid salt.

In an even further preferred embodiment of the invention the reducing agent is a dithionite salt. The reduction can be achieved with sodium dithionite in water according to the method as disclosed by K. Sato, Y. Fujima, A. Yamada Bull. Chem. Soc. Jap. 1968, 41, 442-444.

Hence, preferably the reducing agent is either molecular hydrogen or a transfer hydrogenation agent or a dithionite salt.

### Solvent of the formula (X)

A key element of the present invention is the solvent of formula (X).

Both reaction steps a) and b) are performed in at least one solvent of the formula (X) 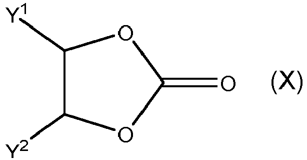
wherein Y¹ and Y² represent independently from each other either H or a methyl or ethyl group;
or in a two-phase solvent mixture comprising at least one solvent (**A**) being of a solvent of the formula (X) and at least one solvent (**B**) which is not miscible with solvent of the formula (X) at room temperature in a volume ratio of (**A**)/(**B**) of between 1/50 and 50/1.

The substituent Y¹ is preferably H.

In a preferred embodiment, Y¹ = Y² = H. Hence, ethylene carbonate is a preferred solvent of the formula (X).

In a further preferred embodiment, Y¹ = H and Y² = CH₃. Hence, propylene carbonate is another preferred solvent of the formula (X).

In a further preferred embodiment, Y¹ = H and Y² = CH₂CH₃. Hence, butylene carbonate is another preferred solvent of the formula (X).

Ethylene carbonate and propylene carbonate are the most preferred solvents of the formula (X).

In a very preferred embodiment, both reaction steps a) and b) are performed in at least two solvents of the formula (X), particularly a binary or ternary mixture of ethylene carbonate and/or propylene carbonate and/or butylene carbonate, most preferably a binary mixture of ethylene carbonate and propylene carbonate. It is preferred that the ratio of ethylene carbonate to propylene carbonate is between 20:80 to 80:20, particularly 25:75 to 75:25.

Preferably, the solvent of the formula is a carbonate solvent as commercialized by Huntsman under the trademark Jeffsol^{®}, particularly the blends of ethylene carbonate with propylene carbonate Jeffsol^{®} EC-75, Jeffsol^{®} EC-50 and Jeffsol^{®} EC-25.

In a further embodiment, the reaction steps a) and/or b) are performed in a two-phase solvent mixture comprising at least one solvent (**A**) being of a solvent of the formula (X) and at least one solvent (**B**) which is not miscible with solvent of the formula (X) at room temperature in a volume ratio of (**A**)/(**B**) of between 1/50 and 50/1.

The solvent (**B**) which is not miscible with the solvent (**A**) of the formula (X) is preferably a hydrocarbon, more preferably an alkane, particularly a C₅₋₁₆-alkane, most particularly a C₆₋₈-alkane. Most preferably the solvent (**B**) is either hexane or heptane.

A "C_{x-y}-alkane" accordingly corresponds to a linear or branched alkane comprising x to y carbon atoms.

It has been shown that the above process allows to oxidize TMP to TMQ as well as to reduce TMQ to TMHQ in an efficient way to the compound of the formula (I). The use of the solvent of formula (X) is very advantageous as the starting material for both processes, i.e. the compound of formula (II), respectively of formula (III), as well as the compound of the formula (I) are well soluble and no precipitation occurs. The good solubility also allows that the process can be performed at low temperatures, particularly at temperatures below 70 °C, more preferably at temperatures between the melting point of the solvent of the formula (X) and 60°C.

The solubility of hydrogen in the solvent of formula (X) is very high, allowing a good yield and selectivity.

The solvent of formula (X) has an excellent stability against the oxidizing agent, particularly against molecular oxygen as well against the reducing agent. And the yield for both reaction step is high.

Furthermore, it has been observed that in said process the solvent of the formula is very stable under the reaction conditions. It has been surprisingly not observed that the solvent is decomposed into the respective diol and CO₂. Furthermore, no formation of ethers between the respective diol and the phenol of the formula (I) have been observed.

After the end of the reaction, the catalyst can be easily removed from the reaction mixture. Particularly useful for this separation are either filtration or extraction.

The process is also very advantageous in that on the one hand the compound of the formula (III) can be prepared in the solvent of the formula (X) and the condensation of the compound of the formula (I) with phytol or isophytol to yield tocopherol on the other hand can be performed in the solvent of the formula (X). This is particularly advantageous as no change of solvent needs to be made during the whole process starting from (poly) alkylphenol to tocopherol.

Alkylene carbonates are very attractive solvents due to their excellent ecotoxicity and positive fire and working hazard properties as well as in view of cost.

The following representation shows the process for the manufacturing of TMHQ from TMP in the alkylene carbonate of formula (X):

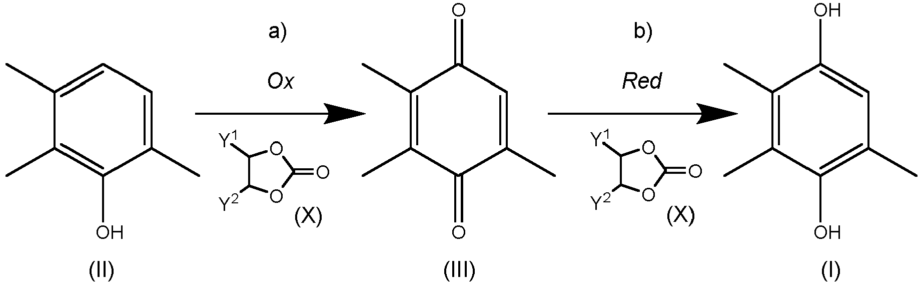

Therefore, in a further embodiment, the invention relates to the use of ethylene carbonate and/or propylene carbonate and/or butylene carbonate as a solvent for the reduction of 2,3,5-trimethyl-1,4-benzoquinone to 2,3,5-trimethylhydroquinone.

### Examples

The present invention is further illustrated by the following experiments.

### Oxidation of TMP

7.5 g CuCl₂·2H₂O, and the respective solvent have been added into the reactor, then 5.9 g melted 2,3,6-trimethylphenol has been added slowly (60 minutes) under stirring with a KBG-stirrer with teflon blade at 80°C and under flowing oxygen (1 Nml/min at a pressure of 1.25 bar). After the TMP has been added stirring continued for another 30 minutes.

The product, i.e. 2,3,5-trimethylquinone (TMQ), has been isolated from the reaction mixture in a yield as given in table 1, and characterized.

The amounts of 4-chloro-2,3,6-trimethylphenol = "CI-TMP" has been determined by GC. The amounts of ethylene glycol have been identified by HPLC with a refraction index detector.

**Table 1. Oxidation of TMP to TMQ in different solvents.**

| | ***1*** | ***Ref.*** |
|---|---|---|
| Ethylene carbonate [g] | 50 | |
| Diethylene glycol monomethyl ether [g] | | 12 |
| Yield [%] | 91.9 | 94.9 |
| CI-TMP [%] | 0.0 | 0.1 |
| Ethylene glycol [%] | 0.0 | 0.0 |

The results of table 1 show that the oxidation in the carbonate can be performed in similarly high yields as in other solvents. However, in the oxidation in carbonates no chlorinated side products (such as CI-TMP) have been found. Also no formation of ethylene glycol has been observed.

### Reduction of TMQ

30.24 g (200 mmol) of 2,3,5-trimethylquinone (TMQ) dissolved in 122 g of the warm solvent as given in table 2 have been added to a 350 ml 4-neck glass flask. 0.048 mol % (0.0934 mmol) Pd/C hydrogenation catalyst (5% metal loading) have been added. After inertisation with nitrogen (3 rinsing/evacuation cycles) molecular hydrogen has been added and heated to 80°C under stirring. After 30 minutes no hydrogen was consumed anymore. The reaction was stirred for another 30 minutes at this temperature.

After removal of the catalyst, a clear solution **A** was obtained without any precipitation. From this solution the formed product, i.e. 2,3,5-trimethylhydroquinone (TMHQ), can be isolated. Such isolation by extraction show a very high yield.

**Table 2: Reduction of TMQ to TMHQ**

| | ***2*** | ***3*** |
|---|---|---|
| Solvent | Propylene carbonate | Ethylene carbonate |
| Yield [%] | 94.5 | 96.0 |

## Claims

1. A process for the manufacturing the compound of the formula (I) comprising the steps reaction step a) followed by reaction step b)
a) oxidation of a compound of the formula (II) to yield a compound of the formula (III); 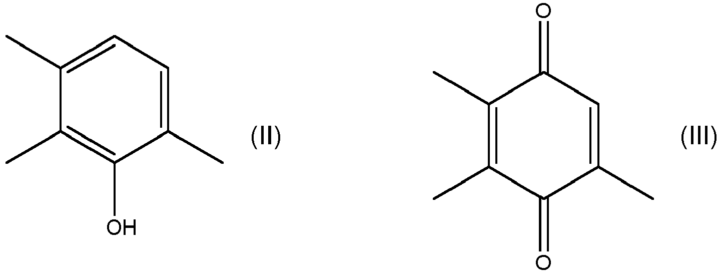
b) reduction of the compound of the formula (III) to yield the compound of the formula (I) 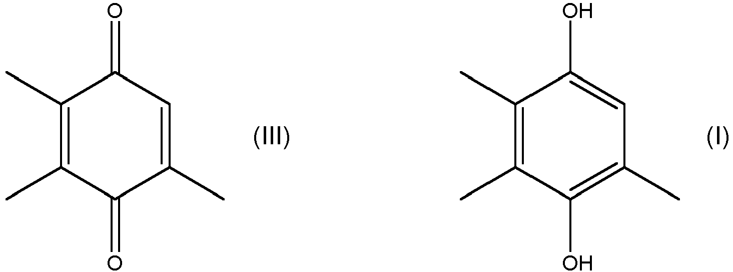 **characterized in that** both reaction steps a) and b) are performed in at least one solvent of the formula (X) 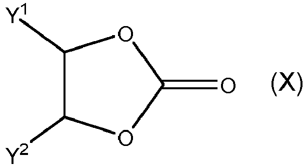 wherein Y¹ and Y² represent independently from each other either H or a methyl or ethyl group;
or in a two-phase solvent mixture comprising at least one solvent (**A**) being of formula (X) and at least one solvent (**B**) which is not miscible with solvent of formula (X) at room temperature in a volume ratio of (**A**)/(**B**) of between 1/50 and 50/1.

2. The process according to claim 1, **characterized in that** Y¹ = Y² = H.

3. The process according to claim 1, **characterized in that** Y¹ = H and Y² = CH₃.

4. The process according to any one of the preceding claims, **characterized in that** that the reaction steps a) and/or b) and the reduction are performed in a solvent mixture of a solvent (**A**) of the formula (X) and a solvent (**B**) being a hydrocarbon, preferably an alkane, more preferably a C₅₋₁₆-alkane, particularly a C₆₋₈-alkane.

5. The process according to anyone of the preceding claims, **characterized in that** both reaction steps a) and b) are performed in a solvent mixture of at least two solvents of formula (X), particularly in a mixture of ethylene carbonate and propylene carbonate.

6. The process according to anyone of the preceding claims **characterized in that** the oxidation in step a) is performed using an oxidizing agent which is either oxygen or hydrogen peroxide, in the presence of a catalyst.

7. The process according to anyone of the preceding claims **characterized in that** the reduction in step b) is performed using a reducing agent which is either molecular hydrogen or a transfer hydrogenation agent or a dithionite salt.

8. The process according to anyone of the preceding claims **characterized in that** the reduction in step b) is performed using a reducing agent which is molecular hydrogen in the presence of a metal catalyst wherein the metal is selected from the group consisting of Ni, Ir, Pd, Pt, Rh and Ru, particularly Pd or Pt, particularly a heterogeneous metal catalyst on a carrier or a support material.

9. The process according to claim 8, **characterized in that** the carrier is a carbon or an oxide of Si, Al, Ce, Ti or Zr, particularly of Al or Si, preferably carbon.

10. The process according to claim 8 or 9, **characterized in that** the catalyst is Pd on carbon.

11. The process according to anyone of the preceding claims claim 8 to 10, **characterized in that** the surface area of the carrier is in the range of 800 to 1500 m²/g, particularly of 1000 to 1200 m²/g.

12. The process according to anyone of the preceding claims 8-11 **characterized in that** the ratio of the weight of the metal, particularly of Pd or Pt, to the weight of the catalyst is between 1 to 20%, preferably between 4 and 11%, more preferably between 4 and 6% by weight.

13. The process according to claim 8, **characterized in that** the support material is a structured packing element.

## Patentansprüche

1. Verfahren zur Herstellung der Verbindung der Formel (I), umfassend die Schritte Reaktionsschritt a) gefolgt von Reaktionsschritt b):
a) Oxidation einer Verbindung der Formel (II) unter Erhalt einer Verbindung der Formel (III); 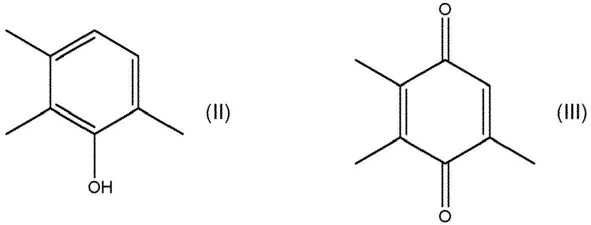
b) Reduktion der Verbindung der Formel (III) unter Erhalt der Verbindung der Formel (I) 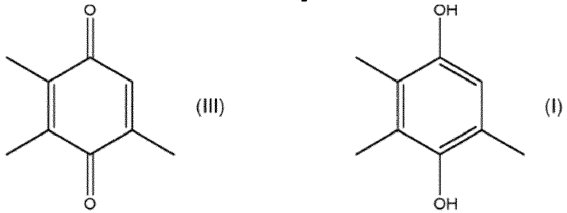 **dadurch gekennzeichnet, dass** beide Reaktionsschritte a) und b) in mindestens einem Lösungsmittel der Formel (X) 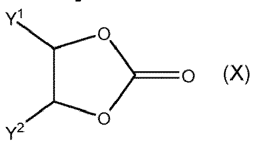
wobei Y¹ oder Y² unabhängig voneinander entweder für H oder eine Methyl- oder Ethylgruppe stehen;
oder in einer zweiphasigen Lösungsmittelmischung, die mindestens ein Lösungsmittel (**A**), das die Formel (X) aufweist, und mindestens ein Lösungsmittel (**B**), das bei Raumtemperatur nicht mit Lösungsmittel der Formel (X) mischbar ist, in einem Volumenverhältnis (**A**)/(**B**) zwischen 1/50 und 50/1 umfasst, durchgeführt werden.

2. Verfahren nach Anspruch 1, **dadurch gekennzeichnet, dass** Y¹ = Y² = H.

3. Verfahren nach Anspruch 1, **dadurch gekennzeichnet, dass** Y¹ = H und Y² = CH₃.

4. Verfahren nach einem der vorhergehenden Ansprüche, **dadurch gekennzeichnet, dass** die Reaktionsschritte a) und/oder b) und die Reduktion in einer Lösungsmittelmischung aus einem Lösungsmittel (**A**) der Formel (X) und einem Lösungsmittel (**B**), bei dem es sich um einen Kohlenwasserstoff, vorzugsweise ein Alkan, weiter bevorzugt ein C₅₋₁₆-Alkan, insbesondere ein C₆₋₈-Alkan, handelt, durchgeführt werden.

5. Verfahren nach einem der vorhergehenden Ansprüche, **dadurch gekennzeichnet, dass** beide Reaktionsschritte a) und b) in einer Lösungsmittelmischung aus mindestens zwei Lösungsmitteln der Formel (X), insbesondere einer Mischung aus Ethylencarbonat und Propylencarbonat, durchgeführt werden.

6. Verfahren nach einem der vorhergehenden Ansprüche, **dadurch gekennzeichnet, dass** die Oxidation in Schritt a) mit einem Oxidationsmittel, bei dem es sich entweder um Sauerstoff oder Wasserstoffperoxid handelt, in Gegenwart eines Katalysators durchgeführt wird.

7. Verfahren nach einem der vorhergehenden Ansprüche, **dadurch gekennzeichnet, dass** die Reduktion in Schritt b) mit einem Reduktionsmittel durchgeführt wird, bei dem es sich entweder um molekularen Wasserstoff oder ein Transferhydrierungsmittel oder ein Dithionitsalz handelt.

8. Verfahren nach einem der vorhergehenden Ansprüche, **dadurch gekennzeichnet, dass** die Reduktion in Schritt b) mit einem Reduktionsmittel, bei dem es sich um molekularen Wasserstoff handelt, in Gegenwart eines Metallkatalysators, wobei das Metall aus der Gruppe bestehend aus Ni, Ir, Pd, Pt, Rh und Ru, insbesondere Pd oder Pt, ausgewählt wird, insbesondere eines heterogenen Metallkatalysators auf einem Träger oder Trägermaterial, durchgeführt wird.

9. Verfahren nach Anspruch 8, **dadurch gekennzeichnet, dass** es sich bei dem Träger um einen Kohlenstoff oder ein Oxid von Si, Al, Ce, Ti oder Zr, insbesondere von Al oder Si, vorzugsweise Kohlenstoff, handelt.

10. Verfahren nach Anspruch 8 oder 9, **dadurch gekennzeichnet, dass** es sich bei dem Katalysator um Pd auf Kohlenstoff handelt.

11. Verfahren nach einem der vorhergehenden Ansprüche 8 bis 10, **dadurch gekennzeichnet, dass** die Oberfläche des Trägers im Bereich von 800 bis 1500 m²/g, insbesondere von 1000 bis 1200 m²/g, liegt.

12. Verfahren nach einem der vorhergehenden Ansprüche 8-11, **dadurch gekennzeichnet, dass** das Verhältnis von Gewicht des Metalls, insbesondere von Pd oder Pt, zu Gewicht des Katalysators zwischen 1 bis 20 Gew.-%, vorzugsweise zwischen 4 und 11 Gew.-%, weiter bevorzugt zwischen 4 und 6 Gew.-%, liegt.

13. Verfahren nach Anspruch 8, **dadurch gekennzeichnet, dass** es sich bei dem Trägermaterial um ein Packungselement handelt.

## Revendications

1. Procédé pour la fabrication du composé de la formule (I) comprenant les étapes étape de réaction a) suivie par étape de réaction b)
a) oxydation d'un composé de la formule (II) pour donner un composé de la formule (III) ; 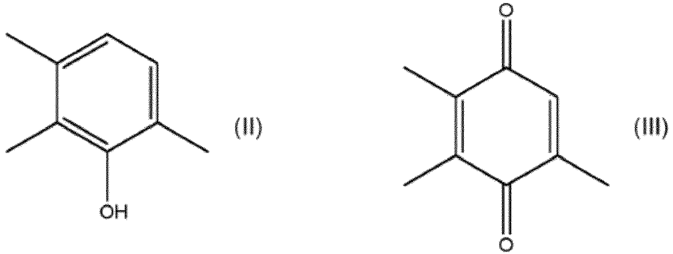
b) réduction du composé de la formule (III) pour donner le composé de la formule (I) 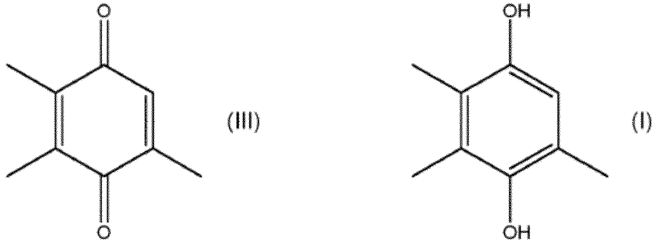 **caractérisé en ce qu'**à la fois les étapes de réaction a) et b) sont réalisées dans au moins un solvant de la formule (X) 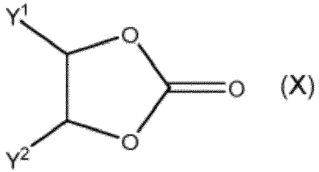 Y¹ et Y² représentant indépendamment l'un de l'autre soit H, soit un groupe méthyle ou éthyle ;
ou dans un mélange de solvants biphasique comprenant au moins un solvant (A) qui est un solvant de la formule (X) et au moins un solvant (B) qui n'est pas miscible avec un solvant de la formule (X) à température ambiante en un rapport volumique de (A)/(B) compris entre 1/50 et 50/1.

2. Procédé selon la revendication 1, **caractérisé en ce que** Y¹ = Y² = H.

3. Procédé selon la revendication 1, **caractérisé en ce que** Y¹ = H et Y² = CH₃.

4. Procédé selon l'une quelconque des revendications précédentes, **caractérisé en ce que** les étapes de réaction a) et/ou b) et la réduction sont réalisées dans un mélange de solvants d'un solvant (A) de la formule (X) et d'un solvant (B) qui est un hydrocarbure, préférablement un alcane, plus préférablement un alcane en C₅₋₁₆, particulièrement un alcane en C₆₋₈.

5. Procédé selon l'une quelconque des revendications précédentes, **caractérisé en ce que** les deux étapes de réaction a) et b) sont réalisées dans un mélange de solvants d'au moins deux solvants de la formule (X), particulièrement dans un mélange de carbonate d'éthylène et de carbonate de propylène.

6. Procédé selon l'une quelconque des revendications précédentes **caractérisé en ce que** l'oxydation dans l'étape a) est réalisée en utilisant un agent oxydant qui est soit l'oxygène, soit le peroxyde d'hydrogène, en la présence d'un catalyseur.

7. Procédé selon l'une quelconque des revendications précédentes, **caractérisé en ce que** la réduction dans l'étape b) est réalisée en utilisant un agent de réduction qui est soit l'hydrogène moléculaire, soit un agent d'hydrogénation par transfert, soit un sel de dithionite.

8. Procédé selon l'une quelconque des revendications précédentes, **caractérisé en ce que** la réduction dans l'étape b) est réalisée en utilisant un agent de réduction qui est l'hydrogène moléculaire en la présence d'un catalyseur métallique, le métal étant choisi dans le groupe constitué par Ni, Ir, Pd, Pt, Rh et Ru, particulièrement Pd ou Pt, particulièrement un catalyseur métallique hétérogène sur un support ou un matériau de support.

9. Procédé selon la revendication 8, **caractérisé en ce que** le support est un carbone ou un oxyde de Si, Al, Ce, Ti ou Zr, particulièrement de Al ou Si, préférablement un carbone.

10. Procédé selon la revendication 8 ou 9, **caractérisé en ce que** le catalyseur est Pd sur carbone.

11. Procédé selon l'une quelconque des revendications précédentes 8 à 10, **caractérisé en ce que** l'aire de surface du support est dans la plage de 800 à 1 500 m²/g, particulièrement de 1 000 à 1 200 m²/g.

12. Procédé selon l'une quelconque des revendications 8 à 11 **caractérisé en ce que** le rapport du poids du métal, particulièrement de Pd ou Pt, sur le poids du catalyseur est compris entre 1 et 20 %, préférablement entre 4 et 11 %, plus préférablement entre 4 et 6 % en poids.

13. Procédé selon la revendication 8, **caractérisé en ce que** le matériau de support est un élément de remplissage structuré.
